# EUROPEAN PATENT APPLICATION

(11) **EP 4 509 062 A1**
(43) Date of publication of application: **19.02.2025**
(21) Application number: 23819250.4
(22) Date of filing: 09.06.2023
(51) Int. Cl.: A61B 10/00

(54) **METHOD FOR PREDICTING FERTILE WINDOW, APPARATUS AND ELECTRONIC DEVICE**

(30) Priority: 10.06.2022 CN 202210658052; 27.09.2022 CN 202211184088
(71) Applicant: Huawei Technologies Co., Ltd., Shenzhen, Guangdong 518129 (CN)
(72) Inventor: HU, Wenfeng, Shenzhen, Guangdong 518129 (CN); LI, Wanyu, Shenzhen, Guangdong 518129 (CN); WANG, Lu, Shenzhen, Guangdong 518129 (CN); SHENG, Rong, Shenzhen, Guangdong 518129 (CN); HE, Zhijian, Shenzhen, Guangdong 518129 (CN)
(74) Representative: Isarpatent
(86) International application number: PCT/CN2023/099312
(87) International publication number: WO 2023/237087

(57) **Abstract**

Embodiments of this application provide a method and an apparatus for predicting a fertile period and an electronic device. In the method for predicting a fertile period, the electronic device obtains physiological parameters of a user and menstruation data entered by the user, and obtains a time length for which the user has worn a wearable device; and if the time length for which the user has worn the wearable device is greater than or equal to a predetermined time length, determines, based on the physiological parameters and the menstruation data entered by the user, whether a current time is within a fertile period, and if the current time is not within the fertile period, determines whether the current time has entered an initial window. If the current time is within the initial window, the electronic device reduces a length of the initial window, and outputs a reduced window. Therefore, as the time length for which the user has worn the wearable device increases, a predicted fertile window of the user can be gradually reduced. This improves accuracy of predicting the fertile window, and improves user experience.

## Description

Embodiments of this application claim priority to Chinese Patent Application No. 202210658052.2, filed with the China National Intellectual Property Administration on June 10, 2022 and entitled "METHOD FOR PREDICTING FERTILE WINDOW", and Chinese Patent Application No. 202211184088.8, filed with the China National Intellectual Property Administration on September 27, 2022 and entitled "METHOD AND APPARATUS FOR PREDICTING FERTILE PERIOD AND ELECTRONIC DEVICE", which are incorporated herein by reference in their entireties.

### TECHNICAL FIELD

Embodiments of this application relate to the field of intelligent terminal technologies, and in particular, to a method and an apparatus for predicting a fertile period and an electronic device.

### BACKGROUND

As female consumers are paying more attention to pregnancy preparation, pregnancy preparation products have come out in the market and become increasingly popular among the consumers. In addition, with ongoing upgrade of mobile internet infrastructure and changes of people's ways of living, an increasing number of women are getting used to using a female physiological health application (application, APP) to record and manage their health conditions. A proportion of female health applications in mobile health applications is increasing every year, reaching nearly 60%. A quantity of users of female physiological health services has exceeded 250 million.

At present, a common method for monitoring fertile periods is a calendar-based method. In the calendar-based method, an ovulation day is calculated based on a menstrual period recorded by a user, to predict a fertile window.

However, the calendar-based method has low prediction accuracy. Most women ovulate at a time prior to about 14 days before next menstruation. However, ovulation is further affected by many other factors such as mood swings, environmental changes, and/or health condition changes, and may be advanced or delayed, resulting in inaccurate prediction of a fertile window.

### SUMMARY

Embodiments of this application provide a method and an apparatus for predicting a fertile period and an electronic device. Embodiments of this application further provide a computer-readable storage medium, to improve accuracy of predicting a fertile window and improve user experience.

According to a first aspect, embodiments of this application provide a method for predicting a fertile period, including: obtaining physiological parameters of a user and menstruation data entered by the user; obtaining a time length for which the user has worn a wearable device; if the time length for which the user has worn the wearable device is greater than or equal to a predetermined time length, determining, based on the physiological parameters and the menstruation data entered by the user, whether a current time is within a fertile period; if the current time is not within the fertile period, determining whether the current time has entered an initial window, where the initial window is predicted based on the menstruation data entered by the user, and a length of the initial window is greater than a predetermined first window length; if the current time is within the initial window, reducing the length of the initial window; and outputting a reduced window.

In the method for predicting a fertile period, an electronic device obtains physiological parameters of a user and menstruation data entered by the user, and obtains a time length for which the user has worn a wearable device; and if the time length for which the user has worn the wearable device is greater than or equal to a predetermined time length, determines, based on the physiological parameters and the menstruation data entered by the user, whether a current time is within a fertile period, and if the current time is not within the fertile period, determines whether the current time has entered an initial window. If the current time is within the initial window, the electronic device reduces a length of the initial window, and outputs a reduced window. Therefore, as the time length for which the user has worn the wearable device increases, a predicted fertile window of the user can be gradually reduced. This improves accuracy of predicting the fertile window, and improves user stickiness and user experience.

In a possible implementation, the reducing the length of the initial window includes: delaying a start time of the initial window.

In a possible implementation, the delaying a start date of the initial window includes: delaying the start time of the initial window by a predetermined unit time length.

In a possible implementation, after the determining whether the current time has entered an initial window, the method further includes: if the current time has not entered the initial window, outputting the initial window.

In a possible implementation, after the determining, based on the physiological parameters and the menstruation data entered by the user, whether a current time is within a fertile period, the method further includes: if the current time is within the fertile period, determining that a start time of a fertile window of a current menstrual period is the current time and a length of the fertile window of the current menstrual period is the predetermined first window length, where the current menstrual period includes a menstrual period to which the current time belongs; and outputting the fertile window of the current menstrual period.

In a possible implementation, after the determining that a start time of a fertile window of a current menstrual period is the current time and a length of the fertile window of the current menstrual period is the predetermined first window length, the method further includes: determining that a length of a fertile window of a next menstrual period is the predetermined first window length.

In a possible implementation, after the obtaining a time length for which the user has worn a wearable device, the method further includes: if the time length for which the user has worn the wearable device is less than the predetermined time length, outputting the initial window.

In a possible implementation, after the obtaining physiological parameters of a user and menstruation data entered by the user, the method further includes: if menstruation data obtained at the current time is different from menstruation data obtained at a previous time, when the time length for which the user has worn the wearable device is greater than or equal to the predetermined time length, predicting, based on the physiological parameters and the menstruation data obtained at the current time, whether the current time is within the fertile period; and if the current time is within the fertile period, determining that a start time of a fertile window of a current menstrual period is the current time and a length of the fertile window of the current menstrual period is the predetermined first window length; or if the current time is not within the fertile period, determining that a start time of a fertile window of a current menstrual period is a time after the current time and a length of the fertile window of the current menstrual period is the predetermined first window length.

In a possible implementation, the method further includes: if the menstruation data obtained at the current time is different from the menstruation data obtained at the previous time, when the time length for which the user has worn the wearable device is less than the predetermined time length, obtaining a length of a fertile window predicted at the previous time; predicting, based on the physiological parameters and the menstruation data obtained at the current time, whether the current time is within the fertile period; and if the current time is within the fertile period, determining that the start time of the fertile window of the current menstrual period is the current time and the length of the fertile window of the current menstrual period is the length of the fertile window predicted at the previous time; or if the current time is not within the fertile period, determining that the start time of the fertile window of the current menstrual period is a time after the current time and the length of the fertile window of the current menstrual period is the length of the fertile window predicted at the previous time.

According to a second aspect, embodiments of this application provide an apparatus for predicting a fertile period. The apparatus is included in an electronic device, and the apparatus has functions of implementing behavior of the electronic device in the first aspect and the possible implementations of the first aspect. The functions may be implemented by hardware, or may be implemented by hardware executing corresponding software. The hardware or software includes one or more modules or units corresponding to the foregoing functions, for example, an obtaining module, a determining module, a judgment module, a reduction module, and an output module.

According to a third aspect, embodiments of this application provide an electronic device, including: one or more processors; a memory; a plurality of applications; and one or more computer programs, where the one or more computer programs are stored in the memory, the one or more computer programs include instructions, and when the instructions are executed by the electronic device, the electronic device is enabled to perform the following steps: obtaining physiological parameters of a user and menstruation data entered by the user; obtaining a time length for which the user has worn a wearable device; if the time length for which the user has worn the wearable device is greater than or equal to a predetermined time length, determining, based on the physiological parameters and the menstruation data entered by the user, whether a current time is within a fertile period; if the current time is not within the fertile period, determining whether the current time has entered an initial window, where the initial window is predicted based on the menstruation data entered by the user, and a length of the initial window is greater than a predetermined first window length; if the current time is within the initial window, reducing the length of the initial window; and outputting a reduced window.

In a possible implementation, the step of reducing the length of the initial window that the electronic device is enabled to perform when the instructions are executed by the electronic device includes: delaying a start time of the initial window.

In a possible implementation, the step of delaying the start time of the initial window that the electronic device is enabled to perform when the instructions are executed by the electronic device includes: delaying the start time of the initial window by a predetermined unit time length.

In a possible implementation, after the step of determining whether the current time has entered the initial window that the electronic device is enabled to perform when the instructions are executed by the electronic device, the following step is further performed: if the current time has not entered the initial window, outputting the initial window.

In a possible implementation, after the step of determining, based on the physiological parameters and the menstruation data entered by the user, whether the current time is within the fertile period that the electronic device is enabled to perform when the instructions are executed by the electronic device, the following steps are further performed: if the current time is within the fertile period, determining that a start time of a fertile window of a current menstrual period is the current time and a length of the fertile window of the current menstrual period is the predetermined first window length, where the current menstrual period includes a menstrual period to which the current time belongs; and outputting the fertile window of the current menstrual period.

In a possible implementation, after the step of determining that the start time of the fertile window of the current menstrual period is the current time and the length of the fertile window of the current menstrual period is the predetermined first window length that the electronic device is enabled to perform when the instructions are executed by the electronic device, the following step is further performed: determining that a length of a fertile window of a next menstrual period is the predetermined first window length.

In a possible implementation, after the step of obtaining the time length for which the user has worn the wearable device that the electronic device is enabled to perform when the instructions are executed by the electronic device, the following step is further performed: if the time length for which the user has worn the wearable device is less than the predetermined time length, outputting the initial window.

In a possible implementation, after the step of obtaining the physiological parameters of the user and the menstruation data entered by the user that the electronic device is enabled to perform when the instructions are executed by the electronic device, the following steps are further performed: if menstruation data obtained at the current time is different from menstruation data obtained at a previous time, when the time length for which the user has worn the wearable device is greater than or equal to the predetermined time length, predicting, based on the physiological parameters and the menstruation data obtained at the current time, whether the current time is within the fertile period; and if the current time is within the fertile period, determining that a start time of a fertile window of a current menstrual period is the current time and a length of the fertile window of the current menstrual period is the predetermined first window length; or if the current time is not within the fertile period, determining that a start time of a fertile window of a current menstrual period is a time after the current time and a length of the fertile window of the current menstrual period is the predetermined first window length.

In a possible implementation, when the instructions are executed by the electronic device, the electronic device is enabled to further perform the following steps: if the menstruation data obtained at the current time is different from the menstruation data obtained at the previous time, when the time length for which the user has worn the wearable device is less than the predetermined time length, obtaining a length of a fertile window predicted at the previous time; predicting, based on the physiological parameters and the menstruation data obtained at the current time, whether the current time is within the fertile period; and if the current time is within the fertile period, determining that the start time of the fertile window of the current menstrual period is the current time and the length of the fertile window of the current menstrual period is the length of the fertile window predicted at the previous time; or if the current time is not within the fertile period, determining that the start time of the fertile window of the current menstrual period is a time after the current time and the length of the fertile window of the current menstrual period is the length of the fertile window predicted at the previous time.

It should be understood that technical solutions in the second and third aspects of the embodiments of this application are consistent with the technical solution in the first aspect of the embodiments of this application, and beneficial effect achieved by the aspects and corresponding feasible implementations are similar. Details are not described again.

According to a fourth aspect, embodiments of this application provide a computer-readable storage medium. The computer-readable storage medium stores a computer program, and when the computer program is run on a computer, the computer is enabled to perform the method provided in the first aspect.

According to a fifth aspect, embodiments of this application provide a computer program. When the computer program is executed by a computer, the computer program is used to perform the method provided in the first aspect.

In a possible design, all or some of the programs in the fifth aspect may be stored in a storage medium packaged with the processor, or some or all of the programs may be stored in a memory not packaged with the processor.

### BRIEF DESCRIPTION OF DRAWINGS

FIG. 1 is a diagram of a structure of an electronic device according to an embodiment of this application;
FIG. 2A, FIG. 2B, and FIG. 2C are diagrams of a system architecture of a method for predicting a fertile period according to an embodiment of this application;
FIG. 3 is a flowchart of a method for predicting a fertile period according to an embodiment of this application;
FIG. 4 is a flowchart of a method for predicting a fertile period according to another embodiment of this application;
FIG. 5 is a flowchart of a method for predicting a fertile period according to still another embodiment of this application;
FIG. 6 is a diagram of a method for predicting a fertile period according to still another embodiment of this application;
FIG. 7 is a diagram of a method for predicting a fertile period according to still another embodiment of this application;
FIG. 8 is a diagram of a method for predicting a fertile period according to still another embodiment of this application;
FIG. 9 is a diagram of a method for predicting a fertile period according to still another embodiment of this application; and
FIG. 10 is a diagram of a structure of an electronic device according to another embodiment of this application.

### DESCRIPTION OF EMBODIMENTS

Terms used in implementations of this application are only used to explain specific embodiments of this application, but are not intended to limit this application.

In a calendar-based method, an ovulation day is calculated based on a menstrual period recorded by a user, to provide a fertile window. The fertile window predicted through the calendar-based method generally includes five days before the ovulation day and four days after the ovulation day, or four days before the ovulation day and two days after the ovulation day. The ovulation day is a day prior to about 14 days before menstrual onset of women.

However, the calendar-based method has low accuracy of predicting a fertile window. Most women ovulate at a time prior to about 14 days before next menstruation. However, the ovulation of women is usually also affected by many other factors such as mood swings, environmental changes, and/or health changes, and may be advanced or delayed, resulting in inaccurate prediction of a fertile window easily.

A wearable device may acquire human physiological parameters, and analyze change patterns of the human physiological parameters in a period, to predict a fertile period. The human physiological parameters include, for example, a heart rate, a body temperature, a skin temperature, a respiratory rate, and/or a deep sleep ratio. A fertile window predicted by the wearable device usually includes a small quantity of days, so that a user perceives a more accurate fertile window.

Embodiments of this application provide a method for predicting a fertile period, to predict a fertile window for a user, assisting a user in preparing for pregnancy more effectively. When the user initially wears a wearable device, an initial window (for example, 10 days) is predicted. As a quantity of days for which the user has worn the wearable device increases, a predicted window is gradually reduced (for example, the predicted window may be gradually reduced to six days), so that the user can experience "the longer the wearing time, the higher the accuracy". This improves user stickiness and user experience.

The method for predicting a fertile period provided in the embodiments of this application may be applied to an electronic device. The electronic device may be a smart mobile phone, a tablet computer, a wearable device, an in-vehicle device, an augmented reality (augmented reality, AR) device/a virtual reality (virtual reality, VR) device, a notebook computer, an ultra-mobile personal computer (ultra-mobile personal computer, UMPC), a netbook, a personal digital assistant (personal digital assistant, PDA), or the like. A specific type of the electronic device is not limited in embodiments of this application.

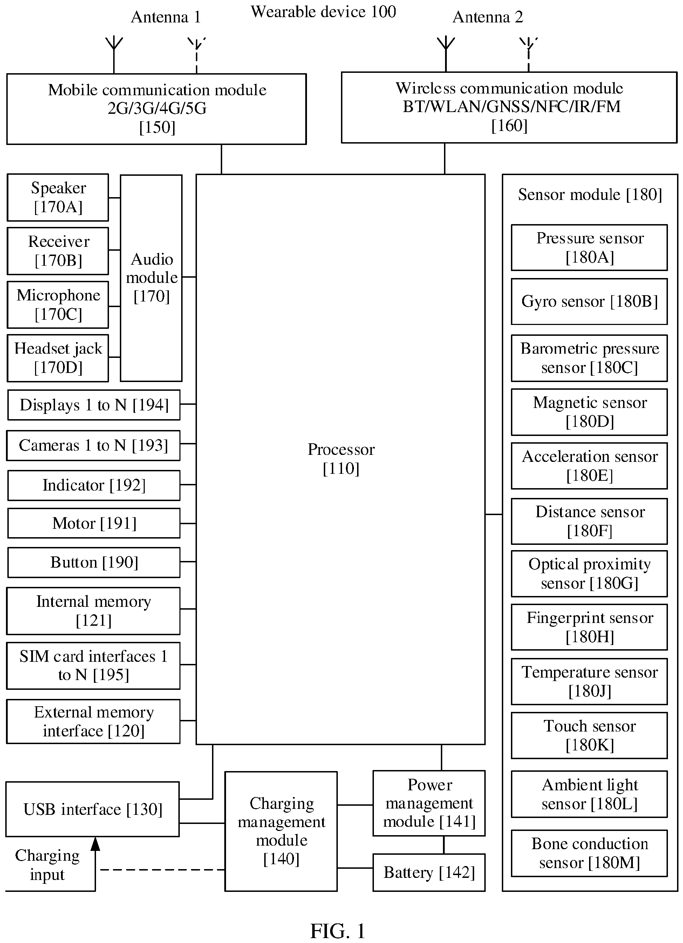

For example, FIG. 1 is a diagram of a structure of an electronic device according to an embodiment of this application. As shown in FIG. 1, the electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that a structure shown in this embodiment of this application does not constitute a specific limitation on the electronic device 100. In other embodiments of this application, the electronic device 100 may include more or fewer components than those shown in the figure, combine some components, split some components, or have different component arrangements. The components shown in the figure may be implemented in hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction fetching and instruction execution.

A memory may be disposed in the processor 110, and is configured to store instructions and data. In some embodiments, the memory in the processor 110 is a cache memory. The memory may store instructions or data that has been used or is cyclically used by the processor 110. If the processor 110 needs to use the instructions or the data again, the processor 110 may directly invoke the instructions or the data from the memory, thereby avoiding repeated access, reducing waiting time of the processor 110, and improving system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transfer an audio signal to the wireless communication module 160 through the I2S interface, to implement a function of answering a call through a Bluetooth headset.

The PCM interface may also be configured to perform audio communication, and sample, quantize, and code an analog signal. In some embodiments, the audio module 170 may be coupled to the wireless communication module 160 through a PCM bus interface. In some embodiments, the audio module 170 may also transfer an audio signal to the wireless communication module 160 through the PCM interface, to implement a function of answering a call through a Bluetooth headset. Both the I2S interface and the PCM interface may be used for audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transfer an audio signal to the wireless communication module 160 through the UART interface, to implement a function of playing music through a Bluetooth headset.

The MIPI interface may be configured to connect the processor 110 to a peripheral component like the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device 100. The processor 110 communicates with the display 194 through the DSI interface, to implement a display function of the electronic device 100.

The GPIO interface may be configured through software. The GPIO interface may be configured as a control signal or may be configured as a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB interface, a micro USB interface, a USB type C interface, or the like. The USB interface 130 may be configured to connect to the charger to charge the electronic device 100, or may be configured to transmit data between the electronic device 100 and a peripheral device, or may be configured to connect to a headset for playing an audio through the headset. The interface may be configured to connect to another wearable device like an AR device.

It can be understood that an interface connection relationship between modules illustrated in this embodiment of this application is merely an illustrative description, and does not constitute a limitation on a structure of the electronic device 100. In some other embodiments of this application, the electronic device 100 may alternatively use an interface connection manner different from an interface connection manner in this embodiment, or use a combination of a plurality of interface connection manners.

The charging management module 140 is configured to receive a charging input from the charger. The charger may be a wireless charger or a wired charger. In some embodiments in which wired charging is used, the charging management module 140 may receive a charging input from the wired charger through the USB interface 130. In some embodiments in which wireless charging is used, the charging management module 140 may receive a wireless charging input through a wireless charging coil of the electronic device 100. While charging the battery 142, the charging management module 140 may further supply power to the electronic device 100 through the power management module 141.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be multiplexed, to improve antenna utilization. For example, the antenna 1 may be multiplexed as a diversity antenna in a wireless local area network. In some other embodiments, an antenna may be used in combination with a tuning switch.

The mobile communication module 150 may provide a solution, applied to the electronic device 100, to wireless communication including 2G, 3G, 4G, 5G, and the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules in the mobile communication module 150 may be disposed in a same device as at least some modules in the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low frequency baseband signal. Then, the demodulator transfers the low-frequency baseband signal obtained through demodulation to the baseband processor for processing. The low-frequency baseband signal is processed by the baseband processor and then transferred to the application processor. The application processor outputs a sound signal by an audio device (which is not limited to the speaker 170A, the receiver 170B, or the like), or displays an image or a video on the display 194. In some embodiments, the modem processor may be an independent device. In some other embodiments, the modem processor may be independent of the processor 110, and is disposed in a same device as the mobile communication module 150 or another functional module.

The wireless communication module 160 may provide a wireless communication solution that is applied to the electronic device 100, and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices integrating at least one communications processing module. The wireless communication module 160 receives an electromagnetic wave through the antenna 2, performs frequency modulation and filtering processing on the electromagnetic wave signal, and sends a processed signal to the processor 110. The wireless communication module 160 may further receive a to-be-sent signal from the processor 110, perform frequency modulation and amplification on the signal, and convert a processed signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device through a wireless communications technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The electronic device 100 implements a display function through the GPU, the display 194, the application processor, and the like. The GPU is a microprocessor for image processing and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, quantum dot light-emitting diode (quantum dot light-emitting diode, QLED), or the like. In some embodiments, the electronic device 100 may include one or N displays 194, where N is a positive integer greater than 1.

The electronic device 100 may implement a photographing function through the ISP, the camera 193, the video codec, the GPU, the display 194, the application processor, and the like.

The ISP is configured to process data fed back by the camera 193. For example, during photographing, a shutter is pressed, and light is transferred to a photosensitive element of the camera through a lens. An optical signal is converted into an electrical signal, and the photosensitive element of the camera transfers the electrical signal to the ISP for processing, to convert the electrical signal into a visible image. The ISP may further perform algorithm optimization on noise, brightness, and complexion of the image. The ISP may further optimize parameters such as exposure and color temperature of a photographing scenario. In some embodiments, the ISP may be disposed in the camera 193.

The camera 193 is configured to capture a still image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for converting the electrical signal into a digital image signal. The ISP outputs the digital image signal to a DSP for processing. The DSP converts the digital image signal into a standard image signal of a format such as RGB or YUV. In some embodiments, the electronic device 100 may include one or N cameras 193, where N is a positive integer greater than 1.

The digital signal processor is configured to process a digital signal, and may process another digital signal in addition to a digital image signal. For example, when the electronic device 100 selects a frequency, the digital signal processor is configured to perform Fourier transform on frequency energy.

The video codec is configured to: compress or decompress a digital video. The electronic device 100 may support one or more video codecs. In this way, the electronic device 100 can play or record videos in a plurality of encoding formats, for example, moving picture experts group (moving picture experts group, MPEG)-1, MPEG-2, MPEG-3, and MPEG-4.

The NPU is a neural-network (neural-network, NN) computing processor, and simulates a biological neural network structure like a transmission mode between neurons in a human brain to perform rapid processing on input information, and can perform continuous self-learning. Applications such as intelligent cognition of the electronic device 100 may be implemented through the NPU, for example, image recognition, facial recognition, speech recognition, and text understanding.

The external memory interface 120 may be configured to connect to an external memory card, for example, a micro SD card, to extend a storage capability of the electronic device 100. The external memory card communicates with the processor 110 through the external memory interface 120, to implement a data storage function. For example, files such as music and a video are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a sound playing function, an image playing function, or the like), and the like. The data storage area may store data (for example, audio data, an address book, or the like) created during use of the electronic device 100, and the like. In addition, the internal memory 121 may include a high-speed random access memory, and may further include a nonvolatile memory, for example, at least one magnetic disk storage device, a flash storage device, and a universal flash storage (universal flash storage, UFS). The processor 110 runs instructions stored in the internal memory 121 and/or instructions stored in the memory disposed in the processor, to perform various functional applications and data processing of the electronic device 100.

The electronic device 100 may implement an audio function such as music playing or recording through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be configured to code and decode an audio signal. In some embodiments, the audio module 170 may be disposed in the processor 110, or some functional modules in the audio module 170 are disposed in the processor 110.

The speaker 170A, also referred to as a "horn", is configured to convert an electrical audio signal into a sound signal. The electronic device 100 may be configured to listen to music or answer a hands-free call through the speaker 170A.

The receiver 170B, also referred to as an "earpiece", is configured to convert an electrical audio signal into a sound signal. When a call is answered or voice information is received through the electronic device 100, the receiver 170B may be put close to a human ear to listen to a voice.

The microphone 170C, also referred to as a "mike" or a "microphone", is configured to convert a sound signal into an electrical signal. When making a call or sending voice information, a user may make a sound near the microphone 170C through the mouth of the user, to input a sound signal to the microphone 170C. The electronic device 100 may be provided with at least one microphone 170C. In some other embodiments, two microphones 170C may be disposed in the electronic device 100, to acquire sound signals and further implement a noise reduction function. In some other embodiments, three, four, or more microphones 170C may alternatively be disposed in the electronic device 100, to acquire a sound signal, implement noise reduction, and identify a sound source, to implement a directional recording function, and the like.

The headset jack 170D is configured to connect to a wired headset. The headset jack 170D may be the USB interface 130, or may be a 3.5 mm open mobile wearable device platform (open mobile terminal platform, OMTP) standard interface or a cellular telecommunications industry association of the USA (cellular telecommunications industry association of the USA, CTIA) standard interface.

The pressure sensor 180A is configured to sense a pressure signal and may convert the pressure signal into an electrical signal. In some embodiments, the pressure sensor 180A may be disposed on the display 194. There are a plurality of types of pressure sensors 180A, for example, a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on a change in the capacitance. When a touch operation is performed on the display 194, the electronic device 100 detects intensity of the touch operation through the pressure sensor 180A. The electronic device 100 may calculate a touch location based on a detection signal of the pressure sensor 180A. In some embodiments, touch operations that are performed at a same touch location but have different touch operation intensity may correspond to different operation instructions. For example, when a touch operation whose touch operation intensity is less than a first pressure threshold is performed on an application icon "Messages", an instruction for viewing an SMS message is executed. When a touch operation whose touch operation intensity is greater than or equal to the first pressure threshold is performed on the application icon "Messages", an instruction for creating an SMS message is executed.

The gyro sensor 180B may be configured to determine a moving posture of the electronic device 100. In some embodiments, an angular velocity of the electronic device 100 around three axes (namely, axes x, y, and z) may be determined through the gyro sensor 180B. The gyro sensor 180B may be configured to implement image stabilization during photographing. For example, when the shutter is pressed, the gyro sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyro sensor 180B may further be used in a navigation scenario and a motion-sensing game scenario.

The barometric pressure sensor 180C is configured to measure barometric pressure. In some embodiments, the electronic device 100 calculates an altitude through a barometric pressure value measured by the barometric pressure sensor 180C, to assist in positioning and navigation.

The magnetic sensor 180D includes a Hall sensor. The electronic device 100 may detect opening and closing of a flip cover leather case through the magnetic sensor 180D. In some embodiments, when the electronic device 100 is a clamshell phone, the electronic device 100 may detect opening and closing of a flip cover based on the magnetic sensor 180D. Further, a feature like automatic unlocking of the flip cover is set based on a detected opening or closing state of the leather case or a detected opening or closing state of the flip cover.

The acceleration sensor 180E may detect the magnitudes of accelerations of the electronic device 100 in various directions (usually on three axes), may detect a magnitude and a direction of gravity when the electronic device 100 is still. The acceleration sensor 180E may be further configured to identify a posture of a wearable device, and is used in an application such as switching between a landscape mode and a portrait mode or a pedometer.

The distance sensor 180F is configured to measure a distance. The electronic device 100 may measure a distance in an infrared manner or a laser manner. In some embodiments, in a shooting scenario, the electronic device 100 may measure a distance through the distance sensor 180F, to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light through the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, through the optical proximity sensor 180G, that a user holds the electronic device 100 close to an ear for a call, to automatically turn off a screen for power saving. The optical proximity sensor 180G may also be used in a leather case mode or a pocket mode to automatically unlock or lock the screen.

The ambient light sensor 180L is configured to sense ambient light brightness. The electronic device 100 may adaptively adjust brightness of the display 194 based on the sensed ambient light brightness. The ambient light sensor 180L may be configured to automatically adjust white balance during photographing. The ambient light sensor 180L may further cooperate with the optical proximity sensor 180G to detect whether the electronic device 100 is in a pocket, to prevent an accidental touch.

The fingerprint sensor 180H is configured to acquire a fingerprint. The electronic device 100 may use a feature of the acquired fingerprint to implement fingerprint-based unlocking, application lock access, fingerprint-based shooting, fingerprint-based call answering, and the like.

The temperature sensor 180J is configured to detect a temperature. In some embodiments, the electronic device 100 executes a temperature processing policy by using the temperature detected by the temperature sensor 180J. For example, when the temperature reported by the temperature sensor 180J exceeds a threshold, the electronic device 100 reduces performance of a processor near the temperature sensor 180J, to reduce power consumption and implement thermal protection. In some other embodiments, when the temperature is less than another threshold, the electronic device 100 heats the battery 142 to prevent the electronic device 100 from being abnormally powered off because of a low temperature. In some other embodiments, when the temperature is less than still another threshold, the electronic device 100 boosts an output voltage of the battery 142, to prevent abnormal power-off caused by a low temperature.

The touch sensor 180K is also referred to as a "touch component". The touch sensor 180K may be disposed on the display 194, and the touch sensor 180K and the display 194 constitute a touchscreen, which is also referred to as a "touchscreen". The touch sensor 180K is configured to detect a touch operation performed on or near the touch sensor 180K. The touch sensor may transfer the detected touch operation to the application processor, to determine a type of a touch event. A visual output related to the touch operation may be provided on the display 194. In some other embodiments, the touch sensor 180K may alternatively be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a human pulse, to receive a blood pressure beating signal. In some embodiments, the bone conduction sensor 180M may alternatively be disposed in the headset, to obtain a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt or a touch vibration feedback. For example, touch operations performed on different applications (for example, photographing and audio playing) may correspond to different vibration feedback effect. The motor 191 may also correspond to different vibration feedback effect for touch operations performed on different areas of the display 194. Different application scenarios (for example, a time reminder, information receiving, an alarm clock, and a game) may also correspond to different vibration feedback effect. Touch vibration feedback effect may be customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a Micro-SIM card, a SIM card, and the like. A plurality of cards may be simultaneously inserted into a same SIM card interface 195. The plurality of cards may be of a same type or of different types. The SIM card interface 195 is compatible to different types of SIM cards. The SIM card interface 195 is also compatible to an external storage card. The electronic device 100 interacts with a network through the SIM card, to implement functions such as talking and data communication. In some embodiments, the electronic device 100 uses an eSIM, that is, an embedded SIM card. The eSIM card may be embedded into the electronic device 100, and cannot be separated from the electronic device 100.

For ease of understanding, in the following embodiments of this application, a wearable device having a structure shown in FIG. 1 is used as an example to specifically describe the method for predicting a fertile period provided in embodiments of this application with reference to the accompanying drawings and application scenarios.

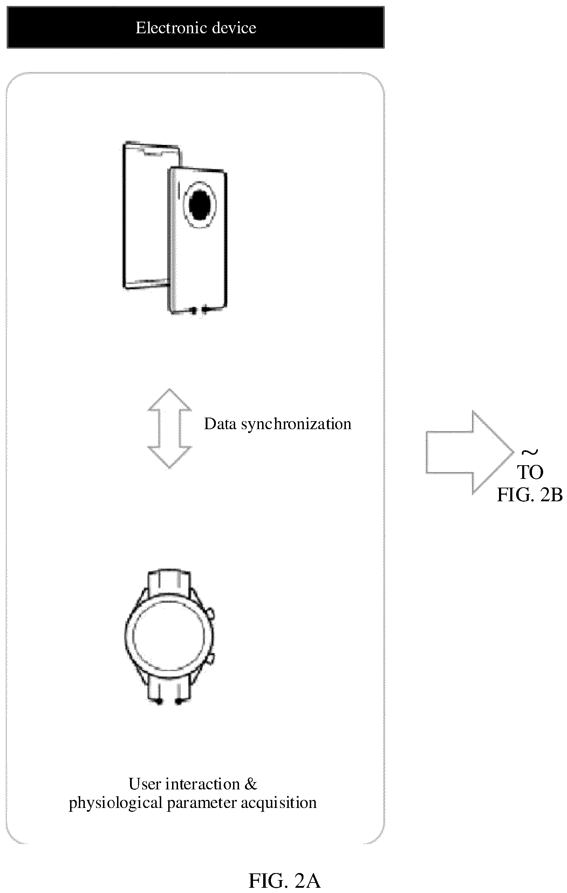

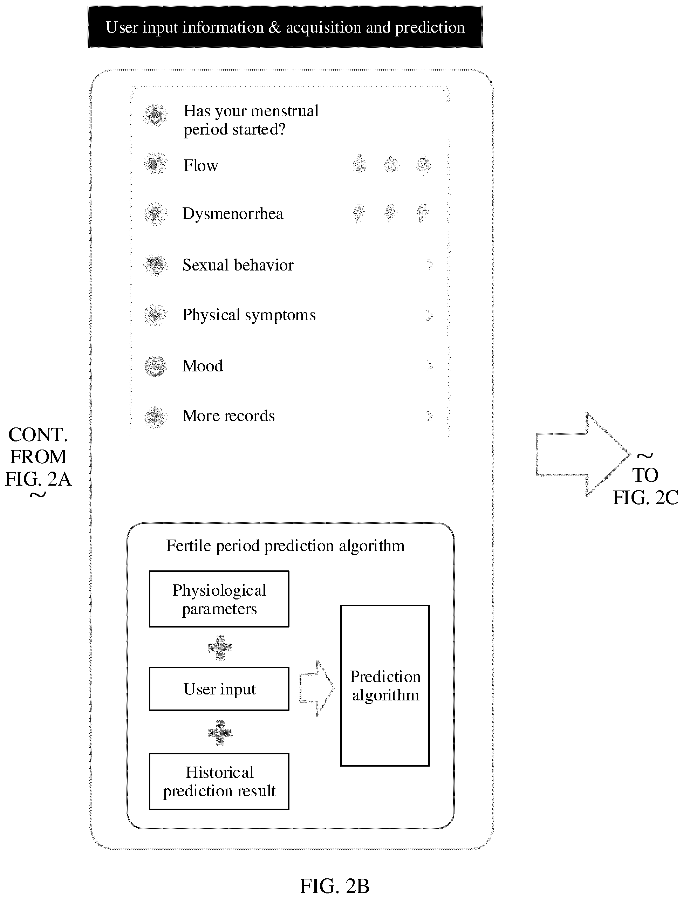

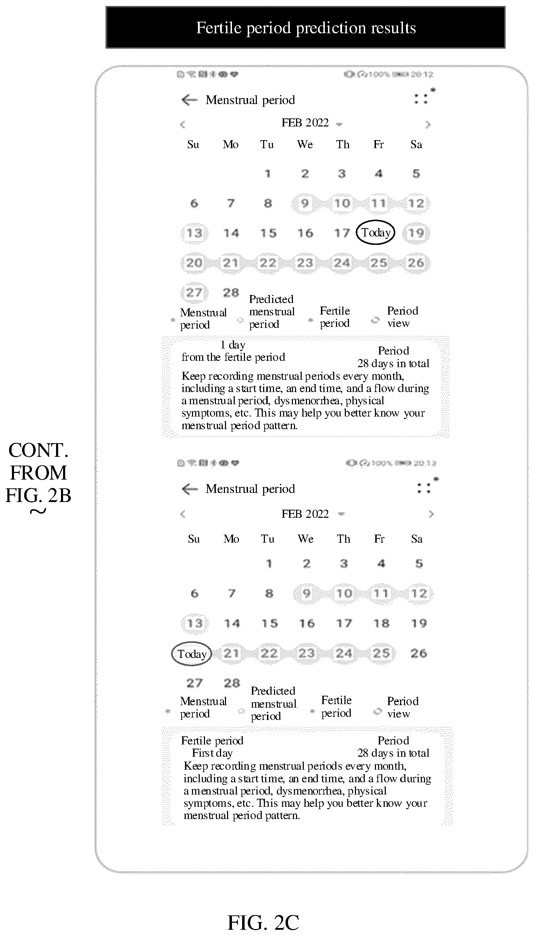

FIG. 2A, FIG. 2B, and FIG. 2C are diagrams of a system architecture of a method for predicting a fertile period according to an embodiment of this application. The foregoing system architecture may include an electronic device 100 (for example, a mobile phone) and a wearable device (for example, a watch). As shown in FIG. 2A, FIG. 2B, and FIG. 2C, a watch is configured to acquire physiological parameters of a user, a female physiological period application runs on a mobile phone, and the mobile phone synchronizes the physiological parameters and prediction data with the watch. Certainly, this is only an implementation. Alternatively, the watch may acquire the physiological parameters of the user, run the female physiological period application, and synchronize the prediction data with the mobile phone. The user may enter a start date and an end date of a menstrual period, physical symptoms, and/or moods, among other information through the female physiological period application. A fertile period prediction algorithm running on the mobile phone may predict a fertile window based on the acquired physiological parameters, the data entered by the user, and historical prediction results. Finally, the mobile phone displays the predicted fertile window through the female physiological period application, and provides a reminder mechanism to remind the user of the start and end of a fertile period.

The following describes, based on the diagram of the system architecture shown in FIG. 2A, FIG. 2B, and FIG. 2C, a method for predicting a fertile period provided in embodiments of this application.

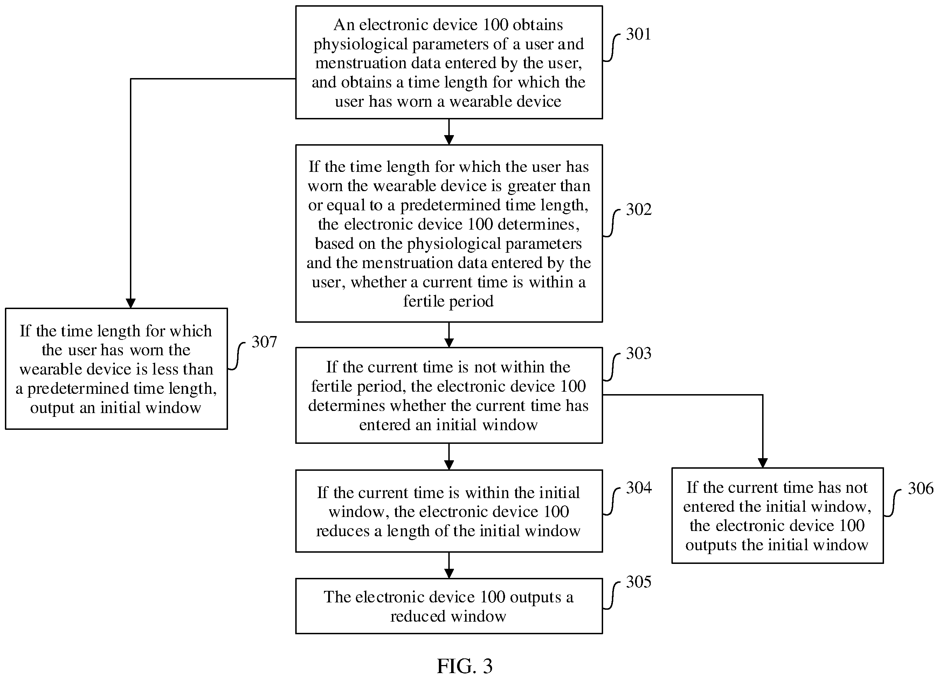

FIG. 3 is a flowchart of a method for predicting a fertile period according to an embodiment of this application. As shown in FIG. 3, the foregoing method for predicting a fertile period may include the following steps.

Step 301: An electronic device 100 obtains physiological parameters of a user and menstruation data entered by the user, and obtains a time length for which the user has worn a wearable device.

Step 302: If the time length for which the user has worn the wearable device is greater than or equal to a predetermined time length, the electronic device 100 determines, based on the physiological parameters and the menstruation data entered by the user, whether a current time is within a fertile period.

The predetermined time length may be set according to system performance, an implementation requirement, and/or the like during specific implementation. For example, the predetermined time length may be 30 days.

Step 303: If the current time is not within the fertile period, the electronic device 100 determines whether the current time has entered an initial window. Then, step 304 or step 306 is performed.

The initial window is within a fertile window predicted by the electronic device 100 based on the menstruation data entered by the user, and a length of the initial window is greater than a predetermined first window length. The predetermined first window length may be set according to system performance, an implementation requirement, and/or the like during specific implementation. A size of the predetermined first window length is not limited in this embodiment. For example, the predetermined first window length may be six days.

Step 304: If the current time is within the initial window, the electronic device 100 reduces the length of the initial window.

Specifically, the reducing the length of the initial window may be: delaying a start time of the initial window. More specifically, the delaying a start time of the initial window may be: delaying the start time of the initial window by a predetermined unit time length.

The predetermined unit time length may be set according to system performance, an implementation requirement, and/or the like during specific implementation. A length of the predetermined unit time length is not limited in this embodiment. For example, the predetermined unit time length may be one day.

Step 305: The electronic device 100 outputs a reduced window.

Specifically, that the electronic device 100 outputs a reduced window may be: the electronic device 100 displays the reduced window; or the electronic device 100 sends the reduced window to another electronic device to be displayed by the another electronic device.

In this embodiment, if the current time is not within the fertile period and the current time is within the initial window, the electronic device 100 delays a start date of the initial window by one day until the fertile window is reduced to the predetermined first window length.

Step 306: If the current time has not entered the initial window, the electronic device 100 outputs the initial window.

In addition, after step 301, the method may further include the following step.

Step 307: If the time length for which the user has worn the wearable device is less than a predetermined time length, output an initial window.

In the method for predicting a fertile period, the electronic device 100 obtains physiological parameters of a user and menstruation data entered by the user, and obtains a time length for which the user has worn a wearable device; and if the time length for which the user has worn the wearable device is greater than or equal to a predetermined time length, the electronic device 100 determines, based on the physiological parameters and the menstruation data entered by the user, whether a current time is within a fertile period, and if the current time is not within the fertile period, the electronic device 100 determines whether the current time has entered an initial window. If the current time is within the initial window, the electronic device 100 reduces a length of the initial window, and outputs a reduced window. Therefore, as the time length for which the user has worn the wearable device increases, a predicted fertile window of the user can be gradually reduced. This improves accuracy of predicting the fertile window, and improves user stickiness and user experience.

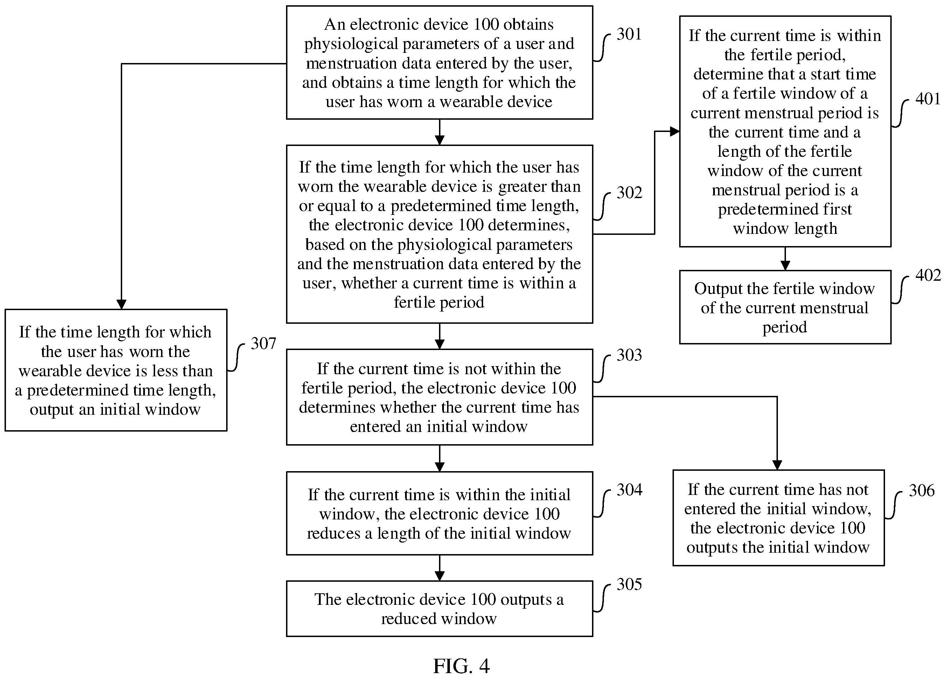

FIG. 4 is a flowchart of a method for predicting a fertile period according to another embodiment of this application. As shown in FIG. 4, in the embodiment shown in FIG. 3 of this application, after step 302, the method may further include the following steps.

Step 401: If the current time is within the fertile period, determine that a start time of a fertile window of a current menstrual period is the current time and a length of the fertile window of the current menstrual period is a predetermined first window length.

The current menstrual period refers to a menstrual period to which the current time belongs.

Specifically, for example, the predetermined first window length is six days. If the electronic device 100 determines, based on the physiological parameters and the menstruation data entered by the user, that the current time is within the fertile period, the electronic device 100 may determine that the fertile window of the current menstrual period is six days starting from a current day.

Step 402: Output the fertile window of the current menstrual period.

Further, after step 401, the electronic device 100 may further determine that a length of a fertile window of a next period is the predetermined first window length.

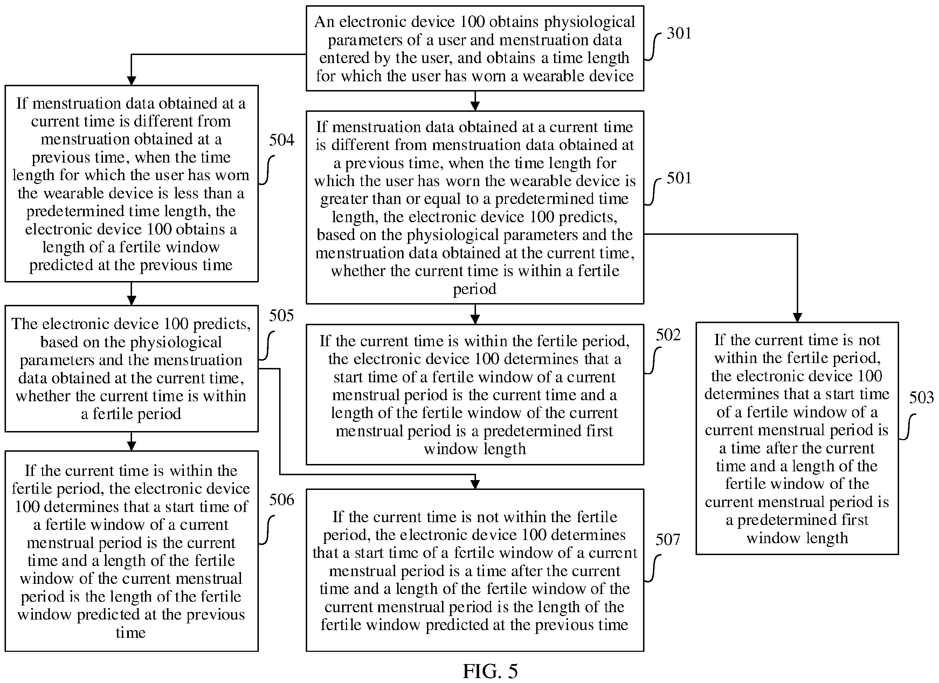

FIG. 5 is a flowchart of a method for predicting a fertile period according to still another embodiment of this application. As shown in FIG. 5, in the embodiment shown in FIG. 3 of this application, after step 301, step 501 or step 504 may be further performed.

Step 501: If menstruation data obtained at a current time is different from menstruation data obtained at a previous time, when the time length for which the user has worn the wearable device is greater than or equal to a predetermined time length, the electronic device 100 predicts, based on the physiological parameters and the menstruation data obtained at the current time, whether the current time is within a fertile period. Then, step 502 or step 503 is performed.

The menstruation data obtained at the previous time may be menstruation data obtained by the electronic device 100 before the current time.

Step 502: If the current time is within the fertile period, the electronic device 100 determines that a start time of a fertile window of a current menstrual period is the current time and a length of the fertile window of the current menstrual period is a predetermined first window length.

Step 503: If the current time is not within the fertile period, the electronic device 100 determines that a start time of a fertile window of a current menstrual period is a time after the current time and a length of the fertile window of the current menstrual period is a predetermined first window length.

For example, the predetermined first window length is still six days. When the time length for which the user has worn the wearable device is greater than or equal to the predetermined time length, if it is predicted, based on the physiological parameters and the menstruation data of the current time, that the current time is within the fertile period, it is determined that the fertile window of the current menstrual period is six days starting from a current day; or if it is predicted that the current time is not within the fertile period, it is determined that the fertile window of the current menstrual period is future six days.

Step 504: If menstruation data obtained at a current time is different from menstruation data obtained at a previous time, when the time length for which the user has worn the wearable device is less than a predetermined time length, the electronic device 100 obtains a length of a fertile window predicted at the previous time.

The length of the fertile window predicted the previous time may be a length of the fertile window predicted by the electronic device 100 before the current time.

Step 505: The electronic device 100 predicts, based on the physiological parameters and the menstruation data obtained at the current time, whether the current time is within a fertile period. Then, step 506 or step 507 is performed.

Step 506: If the current time is within the fertile period, the electronic device 100 determines that a start time of a fertile window of a current menstrual period is the current time and a length of the fertile window of the current menstrual period is the length of the fertile window predicted at the previous time.

Step 507: If the current time is not within the fertile period, the electronic device 100 determines that a start time of a fertile window of a current menstrual period is a time after the current time and a length of the fertile window of the current menstrual period is the length of the fertile window predicted at the previous time.

In this embodiment, the menstruation data obtained at the current time is different from the menstruation data obtained at the previous time, indicating that the user has modified the menstruation data before the current time and after using a female physiological period application installed on the electronic device 100 at the previous time. Simply speaking, if the user has modified the menstruation data before the current time and after using the female physiological period application at the previous time, when the time length for which the user has worn the wearable device is greater than or equal to the predetermined time length, the length of the fertile window predicted by the electronic device 100 is the predetermined first window length; or when the time length for which the user has worn the wearable device is less than the predetermined time length, the length of the fertile window predicted by the electronic device 100 is the same as the length of the fertile window predicted at the previous time.

An example in which an initial window is 10 days and a predetermined first window length is six days is used below to describe a method for predicting a fertile period provided in an embodiment of this application with reference to the accompanying drawings.

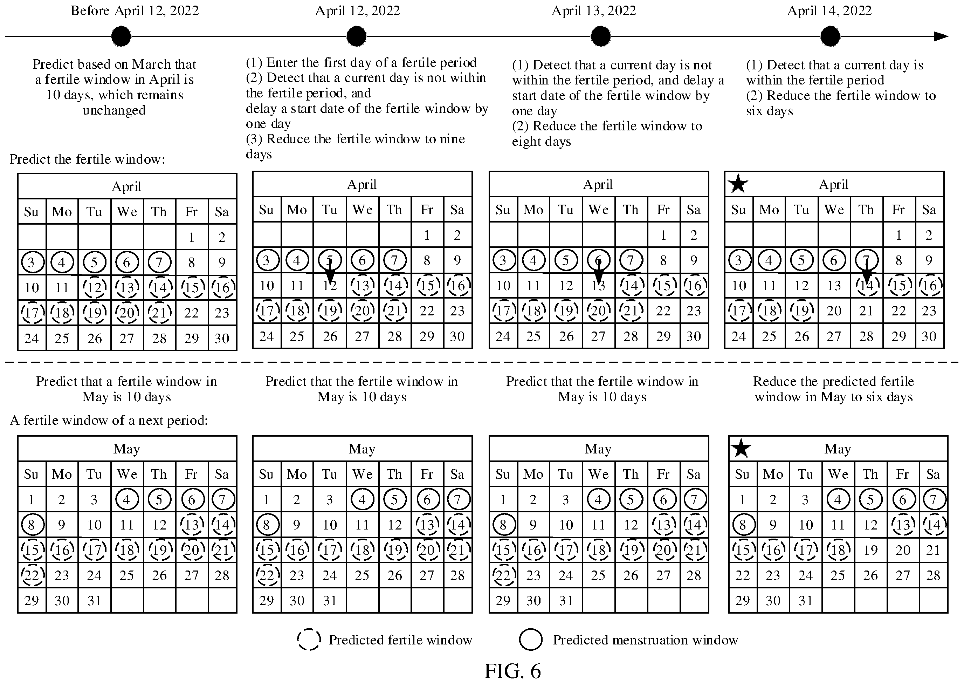

FIG. 6 is a diagram of a method for predicting a fertile period according to still another embodiment of this application. In this embodiment, a current time is a current day, and the following describes a solution in which the current day has entered the 10-day initial window and a predicted fertile window is gradually reduced to six days.

As described above, when a quantity of days for which a user has worn a wearable device is less than a predetermined time length, an electronic device 100 predicts that the initial window is 10 days. After the current day has entered the 10-day initial window, the electronic device 100 reduces the initial window day by day when detecting that the current day is not within a fertile period, and finally reduces the predicted fertile window to six days.

As shown in FIG. 6, before April 12, 2022, because the quantity of days for which the user has worn the wearable device is not large enough, the electronic device 100 predicts that the initial window is 10 days, that is, from April 12 to April 21. In this case, a fertile window in May is also 10 days.

April 12 is the first day of the initial window. The electronic device 100 automatically detects that the current day is not within the fertile period and the quantity of days for which the user has worn the wearable device has reached the predetermined time length. Therefore, a start date of the fertile period is delayed by one day to April 13, and a length of the fertile window is reduced to nine days. In this case, the fertile window in May is still 10 days.

April 13 is the first day of a reduced fertile window. The electronic device 100 automatically detects that the current day is not within the fertile period and the quantity of days for which the user has worn the wearable device has reached the predetermined time length. Therefore, the start date of the fertile period is further delayed by one day to April 14, and the length of the fertile window is reduced to eight days. In this case, the fertile window in May is still 10 days.

April 14 is the first day of a reduced fertile window. The electronic device 100 automatically detects that the current day is within the fertile period and the quantity of days for which the user has worn the wearable device has reached the predetermined time length. Therefore, the length of the fertile window is reduced to six days starting from April 14. In this case, the fertile window in May is also adjusted to six days.

It can be learned from the descriptions that, as the quantity of days for which the user has worn the wearable device increases, the fertile window predicted by the electronic device 100 is gradually reduced from 10 days to six days, so that the user experiences "the longer the wearing time, the higher the accuracy".

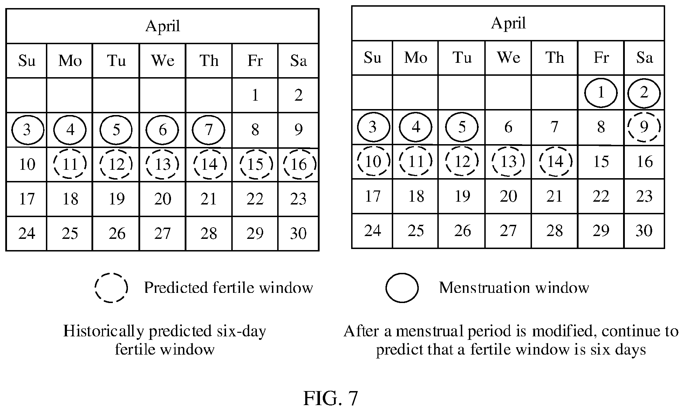

FIG. 7 is a diagram of a method for predicting a fertile period according to still another embodiment of this application. As shown in FIG. 7, if a time length for which a user has worn a wearable device is less than a predetermined time length and a length of a fertile window predicted at a previous time is six days, after menstruation data is modified, it is still predicted that a fertile window is six days. Similarly, if the time length for which the user has worn the wearable device is less than the predetermined time length and the length of the fertile window predicted at the previous time is 10 days, after the menstruation data is modified, it is still predicted that a fertile window is 10 days.

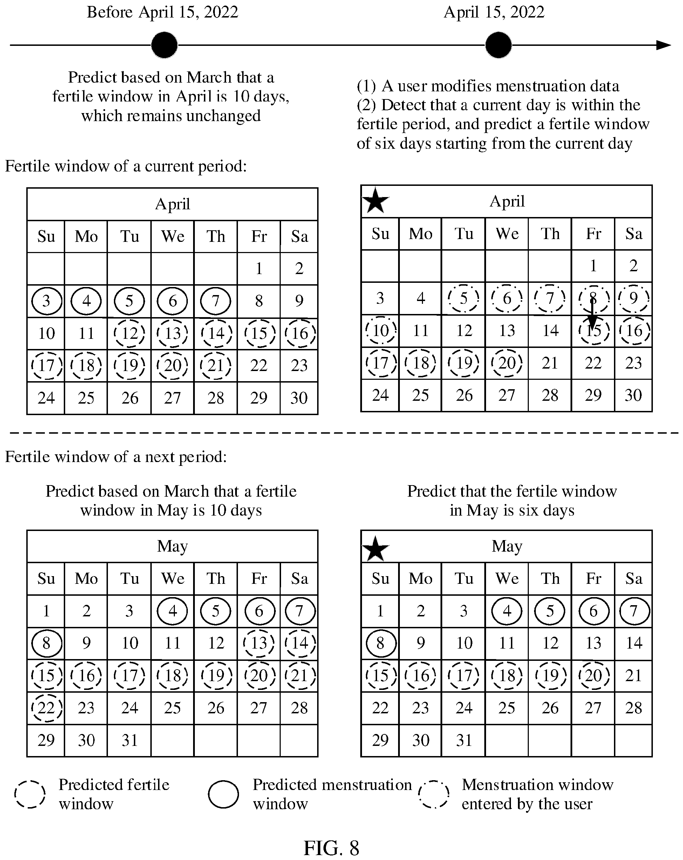

FIG. 8 is a diagram of a method for predicting a fertile period according to still another embodiment of this application. As shown in FIG. 8, if a time length for which a user has worn a wearable device is greater than or equal to a predetermined time length, the user modifies a menstrual period on a current day, and an electronic device 100 detects that the current day is within a fertile period, a predicted fertile window is six days starting from the current day.

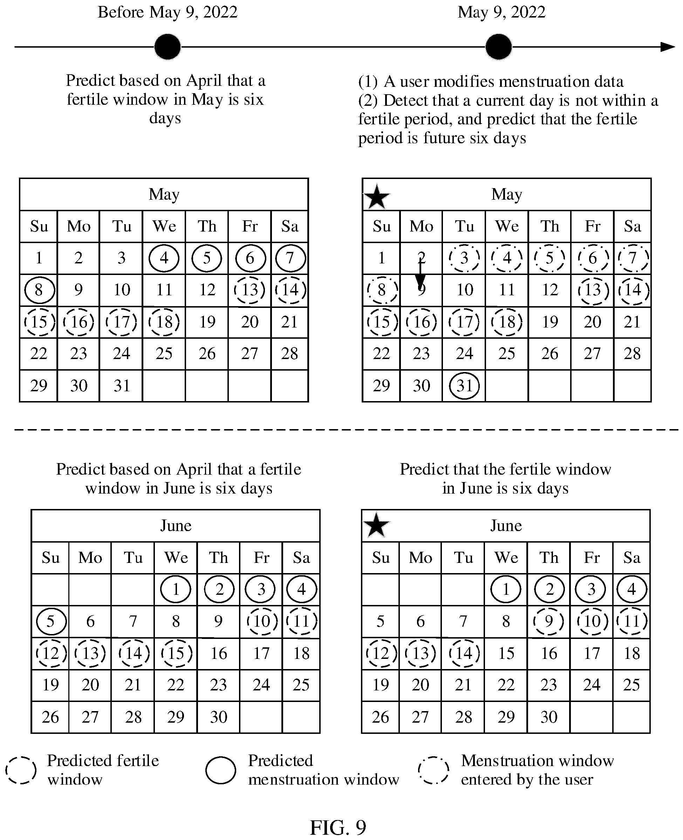

FIG. 9 is a diagram of a method for predicting a fertile period according to still another embodiment of this application. As shown in FIG. 9, if a time length for which a user has worn a wearable device is greater than or equal to a predetermined time length, the user modifies a menstrual period on a current day, and an electronic device 100 detects that the current day is not within a fertile period, a predicted fertile window is future six days.

It can be learned from FIG. 7 to FIG. 9 that, in the prediction method by combining a quantity of days of a historically predicted fertile window predicted historically, a time length for which a user has worn a wearable device, and physiological data according to embodiments of this application, when the user modifies a menstrual period, a sudden change in a length of a fertile window can be avoided, so that user experience is improved.

It can be understood that some of or all of the steps or operations in the foregoing embodiments are merely examples, and other operations or variants of various operations may be further performed in this embodiment of this application. In addition, the steps may be performed in another order different from that presented in the foregoing embodiments, and not all the operations in the foregoing embodiments may be performed.

It may be understood that, to implement the foregoing functions, the electronic device includes corresponding hardware and/or software modules for performing the functions. Algorithm steps in the examples described with reference to embodiments disclosed in this application can be implemented by hardware or a combination of hardware and computer software in this application. Whether a function is performed by hardware or hardware driven by computer software depends on particular applications and design constraints of the technical solutions. A person skilled in the art may use different methods to implement the described functions for each specific application with reference to embodiments. However, it should not be considered that the implementation goes beyond the scope of this application.

In this embodiment, the electronic device may be divided into functional modules according to the foregoing method embodiments. For example, each functional module may be obtained through division based on each corresponding function, or two or more functions may be integrated into one module. The integrated module may be implemented in a form of hardware. It should be noted that, in this embodiment, the division of the modules is an example, and is merely logical function division. During an actual implementation, another division manner may be used.

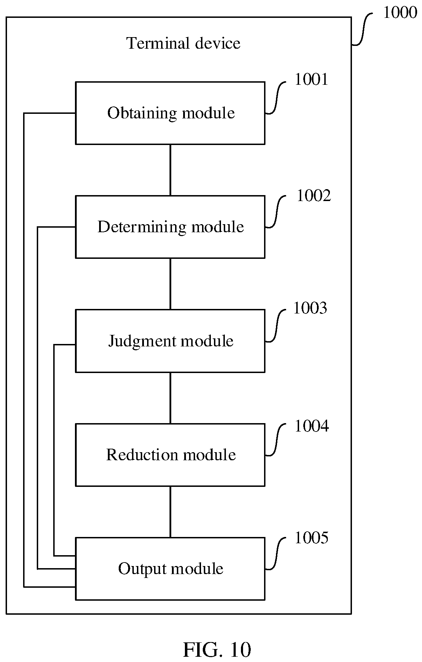

FIG. 10 is a diagram of a structure of an electronic device according to another embodiment of this application. When functional modules are obtained through division based on corresponding functions, FIG. 10 is a possible diagram of a composition of an electronic device 1000 in the foregoing embodiment. As shown in FIG. 10, the electronic device 1000 may include an obtaining module 1001, a determining module 1002, a judgment module 1003, a reduction module 1004, and an output module 1005.

The obtaining module 1001 is configured to: obtain physiological parameters of a user and menstruation data entered by the user; and obtain a time length for which the user has worn a wearable device.

The determining module 1002 is configured to: when the time length for which the user has worn the wearable device is greater than or equal to a predetermined time length, determine, based on the physiological parameters and the menstruation data entered by the user, whether a current time is within a fertile period.

The judgment module 1003 is configured to: when the current time is not within the fertile period, determine whether the current time has entered an initial window, where the initial window is predicted based on the menstruation data entered by the user, and a length of the initial window is greater than a predetermined first window length.

The reduction module 1004 is configured to: when the current time is within the initial window, reduce the length of the initial window. Specifically, the reduction module 1004 is specifically configured to delay a start time of the initial window. More specifically, the reduction module 1004 is specifically configured to delay the start time of the initial window by a predetermined unit time length.

The output module 1005 is configured to output a reduced window.

Further, the output module 1005 is further configured to: after the judgment module 1003 determines whether the current time has entered the initial window, if the current time has not entered the initial window, output the initial window.

The determining module 1002 is further configured to: after determining whether the current time is within the fertile period, if the current time is within the fertile period, determine that a start time of a fertile window of a current menstrual period is the current time and a length of the fertile window of the current menstrual period is the predetermined first window length, where the current menstrual period includes a menstrual period to which the current time belongs.

The output module 1005 is further configured to output the fertile window of the current menstrual period.

Further, the determining module 1002 is further configured to: after determining that the start time of the fertile window of the current menstrual period is the current time and the length of the fertile window of the current menstrual period is the predetermined first window length, determine that a length of a fertile window of a next menstrual period is the predetermined first window length.

Further, the output module 1005 is further configured to: after the obtaining module 1001 obtains the time length for which the user has worn the wearable device, if the time length for which the user has worn the wearable device is less than the predetermined time length, output the initial window.

Further, in this embodiment, the determining module 1002 is further configured to: after the obtaining module 1001 obtains the physiological parameters of the user and the menstruation data entered by the user, if menstruation data obtained at the current time is different from menstruation data obtained at a previous time, when the time length for which the user has worn the wearable device is greater than or equal to the predetermined time length, predict, based on the physiological parameters and the menstruation data obtained at the current time, whether the current time is within the fertile period; and if the current time is within the fertile period, determine that a start time of a fertile window of a current menstrual period is the current time and a length of the fertile window of the current menstrual period is the predetermined first window length; or if the current time is not within the fertile period, determine that a start time of a fertile window of a current menstrual period is a time after the current time and a length of the fertile window of the current menstrual period is the predetermined first window length.

In addition, the obtaining module 1001 is further configured to: when the menstruation data obtained at the current time is different from the menstruation data obtained at the previous time, if the time length for which the user has worn the wearable device is less than the predetermined time length, obtain a length of a fertile window predicted at the previous time.

The determining module 1002 is further configured to: predict, based on the physiological parameters and the menstruation data obtained at the current time, whether the current time is within the fertile period; and if the current time is within the fertile period, determine that the start time of the fertile window of the current menstrual period is the current time and the length of the fertile window of the current menstrual period is the length of the fertile window predicted at the previous time; or if the current time is not within the fertile period, determine that the start time of the fertile window of the current menstrual period is a time after the current time and the length of the fertile window of the current menstrual period is the length of the fertile window predicted at the previous time.

It should be noted that all related content of the steps in the foregoing method embodiments may be cited in function description of corresponding functional modules. Details are not described herein.

The electronic device 1000 provided in this embodiment is configured to perform the methods for predicting a fertile period provided in the embodiments shown in FIG. 3 to FIG. 5 of this application, and therefore can achieve same effect as the foregoing methods.

It should be understood that the electronic device 1000 may correspond to the electronic device shown in FIG. 1. Functions of the obtaining module 1001, the determining module 1002, the judgment module 1003, and the reduction module 1004 may be implemented by the processor 110 in the electronic device shown in FIG. 1, and the function of the output module 1005 may be implemented by the processor 110 and the display 194 in the electronic device shown in FIG. 1.

When an integrated unit is used, the electronic device 1000 may include a processing module, a storage module, and a communication module.

The processing module may be configured to control and manage an action of the electronic device 1000. For example, the processing module may be configured to support the electronic device 1000 in performing the steps performed by the obtaining module 1001, the determining module 1002, the judgment module 1003, the reduction module 1004, and the output module 1005. The storage module may be configured to support the electronic device 1000 in storing program code, data, and the like. The communication module may be configured to support communication between the electronic device 1000 and another device.

The processing module may be a processor or a controller, and may implement or execute various example logic blocks, modules, and circuits described with reference to content disclosed in this application. The processor may alternatively be a combination for implementing a computing function, for example, a combination including one or more microprocessors or a combination of a digital signal processor (digital signal processor, DSP) and a microprocessor. The storage module may be a memory. The communication module may be specifically a device, for example, a radio frequency circuit, a Bluetooth chip, and/or a Wi-Fi chip, that interacts with another electronic device.

In an embodiment, when the processing module is a processor and the storage module is a memory, the electronic device 1000 in this embodiment may be a device with the structure shown in FIG. 1.

An embodiment of this application further provides a computer-readable storage medium. The computer-readable storage medium stores a computer program. When the computer program runs on a computer, the computer is enabled to perform the methods provided in the embodiments shown in FIG. 3 to FIG. 5 of this application.

An embodiment of this application further provides a computer program product. The computer program product includes a computer program. When the computer program runs on a computer, the computer is enabled to perform the methods provided in the embodiments shown in FIG. 3 to FIG. 5 of this application.

In embodiments of this application, "at least one" means one or more, and "a plurality of" means two or more. The term "and/or" describes an association relationship for describing associated objects and represents that three relationships may exist. For example, A and/or B may represent the following three cases: Only A exists, both A and B exist, and only B exists. A and B may be singular or plural. The character "/" generally indicates an "or" relationship between the associated objects. "At least one of the following" and similar expressions refer to any combination of these terms, including any combination of single or plural terms. For example, at least one of a, b, or c may indicate a, b, c, a and b, a and c, b and c, or a, b, and c, where a, b, and c may be singular or plural.

A person of ordinary skill in the art may be aware that the units and algorithm steps described in the embodiments disclosed in this specification can be implemented by a combination of electronic hardware, computer software, and electronic hardware. Whether the functions are performed by hardware or software depends on particular applications and design constraints of the technical solutions. A skilled person may use different methods to implement the described functions for each particular application, but it should not be considered that the implementation goes beyond the scope of this application.

It may be clearly understood by a person skilled in the art that, for the purpose of convenient and brief descriptions, for a detailed working process of the foregoing system, apparatus, and unit, refer to a corresponding process in the foregoing method embodiments. Details are not described herein again.

In several embodiments provided in this application, when any function is implemented in a form of a software functional unit and sold or used as an independent product, the function may be stored on a computer-readable storage medium. Based on such an understanding, the technical solutions of this application essentially, or the part contributing to the current technology, or some of the technical solutions may be implemented in a form of a software product. The computer software product is stored in a storage medium, and includes several instructions for indicating a computer device (which may be a personal computer, a server, or a network device) to perform all or a part of the steps of the methods described in embodiments of this application. The foregoing storage medium includes any medium that can store program code such as a USB flash drive, a removable hard disk, a read-only memory (read-only memory, ROM), a random access memory (random access memory, RAM), a magnetic disk, or an optical disc.

The foregoing descriptions are merely specific implementations of embodiments of this application. Any variation or replacement readily figured out by a person skilled in the art within the technical scope disclosed in this application shall fall within the protection scope in embodiments of this application. The protection scope of this application shall be subject to the protection scope of the claims.

## Claims

1. A method for predicting a fertile period, comprising:
obtaining physiological parameters of a user and menstruation data entered by the user;
obtaining a time length for which the user has worn a wearable device;
if the time length for which the user has worn the wearable device is greater than or equal to a predetermined time length, determining, based on the physiological parameters and the menstruation data entered by the user, whether a current time is within a fertile period;
if the current time is not within the fertile period, determining whether the current time has entered an initial window, wherein the initial window is predicted based on the menstruation data entered by the user, and a length of the initial window is greater than a predetermined first window length;
if the current time is within the initial window, reducing the length of the initial window; and
outputting a reduced window.

2. The method according to claim 1, wherein the reducing the length of the initial window comprises:
delaying a start time of the initial window.

3. The method according to claim 2, wherein the delaying a start date of the initial window comprises:
delaying the start time of the initial window by a predetermined unit time length.

4. The method according to claim 1, wherein after the determining whether the current time has entered an initial window, the method further comprises:
if the current time has not entered the initial window, outputting the initial window.

5. The method according to claim 1, wherein after the determining, based on the physiological parameters and the menstruation data entered by the user, whether a current time is within a fertile period, the method further comprises:
if the current time is within the fertile period, determining that a start time of a fertile window of a current menstrual period is the current time and a length of the fertile window of the current menstrual period is the predetermined first window length, wherein the current menstrual period comprises a menstrual period to which the current time belongs; and
outputting the fertile window of the current menstrual period.

6. The method according to claim 5, wherein after the determining that a start time of a fertile window of a current menstrual period is the current time and a length of the fertile window of the current menstrual period is the predetermined first window length, the method further comprises:
determining that a length of a fertile window of a next menstrual period is the predetermined first window length.

7. The method according to claim 1, wherein after the obtaining a time length for which the user has worn a wearable device, the method further comprises:
if the time length for which the user has worn the wearable device is less than the predetermined time length, outputting the initial window.

8. The method according to claim 1, wherein after the obtaining physiological parameters of a user and menstruation data entered by the user, the method further comprises:
if menstruation data obtained at the current time is different from menstruation data obtained at a previous time, when the time length for which the user has worn the wearable device is greater than or equal to the predetermined time length, predicting, based on the physiological parameters and the menstruation data obtained at the current time, whether the current time is within the fertile period; and
if the current time is within the fertile period, determining that a start time of a fertile window of a current menstrual period is the current time and a length of the fertile window of the current menstrual period is the predetermined first window length; or
if the current time is not within the fertile period, determining that a start time of a fertile window of a current menstrual period is a time after the current time and a length of the fertile window of the current menstrual period is the predetermined first window length.

9. The method according to claim 8, further comprising:
if the menstruation data obtained at the current time is different from the menstruation data obtained at the previous time, when the time length for which the user has worn the wearable device is less than the predetermined time length, obtaining a length of a fertile window predicted at the previous time;
predicting, based on the physiological parameters and the menstruation data obtained at the current time, whether the current time is within the fertile period; and
if the current time is within the fertile period, determining that the start time of the fertile window of the current menstrual period is the current time and the length of the fertile window of the current menstrual period is the length of the fertile window predicted at the previous time; or
if the current time is not within the fertile period, determining that the start time of the fertile window of the current menstrual period is a time after the current time and the length of the fertile window of the current menstrual period is the length of the fertile window predicted at the previous time.

10. An apparatus for predicting a fertile period, comprising:
an obtaining module, configured to: obtain physiological parameters of a user and menstruation data entered by the user; and obtain a time length for which the user has worn a wearable device;
a determining module, configured to: when the time length for which the user has worn the wearable device is greater than or equal to a predetermined time length, determine, based on the physiological parameters and the menstruation data entered by the user, whether a current time is within a fertile period;
a judgment module, configured to: when the current time is not within the fertile period, determine whether the current time has entered an initial window, wherein the initial window is predicted based on the menstruation data entered by the user, and a length of the initial window is greater than a predetermined first window length;
a reduction module, configured to: when the current time is within the initial window, reduce the length of the initial window; and
an output module, configured to output a reduced window.

11. An electronic device, comprising:
one or more processors, a memory, a plurality of applications, and one or more computer programs, wherein the one or more computer programs are stored in the memory, the one or more computer programs comprise instructions, and when the instructions are executed by the electronic device, the electronic device is enabled to perform the following steps:
obtaining physiological parameters of a user and menstruation data entered by the user;
obtaining a time length for which the user has worn a wearable device;
if the time length for which the user has worn the wearable device is greater than or equal to a predetermined time length, determining, based on the physiological parameters and the menstruation data entered by the user, whether a current time is within a fertile period;
if the current time is not within the fertile period, determining whether the current time has entered an initial window, wherein the initial window is predicted based on the menstruation data entered by the user, and a length of the initial window is greater than a predetermined first window length;
if the current time is within the initial window, reducing the length of the initial window; and
outputting a reduced window.

12. The electronic device according to claim 11, wherein the step of reducing the length of the initial window that the electronic device is enabled to perform when the instructions are executed by the electronic device comprises:
delaying a start time of the initial window.

13. The electronic device according to claim 12, wherein the step of delaying the start time of the initial window that the electronic device is enabled to perform when the instructions are executed by the electronic device comprises:
delaying the start time of the initial window by a predetermined unit time length.

14. The electronic device according to claim 11, wherein after the step of determining whether the current time has entered the initial window that the electronic device is enabled to perform when the instructions are executed by the electronic device, the following step is further performed:
if the current time has not entered the initial window, outputting the initial window.

15. The electronic device according to claim 11, wherein after the step of determining, based on the physiological parameters and the menstruation data entered by the user, whether the current time is within the fertile period that the electronic device is enabled to perform when the instructions are executed by the electronic device, the following steps are further performed:
if the current time is within the fertile period, determining that a start time of a fertile window of a current menstrual period is the current time and a length of the fertile window of the current menstrual period is the predetermined first window length, wherein the current menstrual period comprises a menstrual period to which the current time belongs; and
outputting the fertile window of the current menstrual period.

16. The electronic device according to claim 15, wherein after the step of determining that the start time of the fertile window of the current menstrual period is the current time and the length of the fertile window of the current menstrual period is the predetermined first window length that the electronic device is enabled to perform when the instructions are executed by the electronic device, the following step is further performed:
determining that a length of a fertile window of a next menstrual period is the predetermined first window length.

17. The electronic device according to claim 11, wherein after the step of obtaining the time length for which the user has worn the wearable device that the electronic device is enabled to perform when the instructions are executed by the electronic device, the following step is further performed:
if the time length for which the user has worn the wearable device is less than the predetermined time length, outputting the initial window.

18. The electronic device according to claim 11, wherein after the step of obtaining the physiological parameters of the user and the menstruation data entered by the user that the electronic device is enabled to perform when the instructions are executed by the electronic device, the following steps are further performed:
if menstruation data obtained at the current time is different from menstruation data obtained at a previous time, when the time length for which the user has worn the wearable device is greater than or equal to the predetermined time length, predicting, based on the physiological parameters and the menstruation data obtained at the current time, whether the current time is within the fertile period; and
if the current time is within the fertile period, determining that a start time of a fertile window of a current menstrual period is the current time and a length of the fertile window of the current menstrual period is the predetermined first window length; or
if the current time is not within the fertile period, determining that a start time of a fertile window of a current menstrual period is a time after the current time and a length of the fertile window of the current menstrual period is the predetermined first window length.

19. The electronic device according to claim 18, wherein when the instructions are executed by the electronic device, the electronic device is enabled to further perform the following steps:
if the menstruation data obtained at the current time is different from the menstruation data obtained at the previous time, when the time length for which the user has worn the wearable device is less than the predetermined time length, obtaining a length of a fertile window predicted at the previous time;
predicting, based on the physiological parameters and the menstruation data obtained at the current time, whether the current time is within the fertile period; and
if the current time is within the fertile period, determining that the start time of the fertile window of the current menstrual period is the current time and the length of the fertile window of the current menstrual period is the length of the fertile window predicted at the previous time; or
if the current time is not within the fertile period, determining that the start time of the fertile window of the current menstrual period is a time after the current time and the length of the fertile window of the current menstrual period is the length of the fertile window predicted at the previous time.

20. A computer-readable storage medium, wherein the computer-readable storage medium stores a computer program, and when the computer program is run on a computer, the computer is enabled to perform the method according to any one of claims 1 to 9.
